# EUROPÄISCHE PATENTSCHRIFT

(11) **EP 2 792 237 B1**
(45) Veröffentlichungstag und Bekanntmachung des Hinweises auf die Patenterteilung: **03.06.2015**
(21) Anmeldenummer: 13164156.5
(22) Anmeldetag: 17.04.2013
(51) Int. Cl.: A01K 67/033

(54) **Zusammenstellung, Aufzuchtanordnung und Verfahren zur Aufzucht von Spathius exarator**
Composition, breeding assembly and method for breeding Spathius exarator
Composition, dispositif d'élevage et procédé d'élevage de Spathius exarator

(43) Veröffentlichungstag der Anmeldung: 22.10.2014
(73) Patentinhaber: APC AG, 90482 Nürnberg (DE)
(72) Erfinder: Kassel, Alexander, 91238 Offenhausen (DE); Auer, Judith, 91413 Neustadt (DE)
(74) Vertreter: Lippert, Markus Alfons

(56) Entgegenhaltungen:
- EP-A1- 1 161 863
- EP-A1- 1 733 617
- US-A- 3 786 594

## Beschreibung

Die Erfindung betrifft eine Zusammenstellung zur Aufzucht von *Spathius exarator,* eine Aufzuchtanordnung mit einer solchen Zusammenstellung und ein Verfahren zur Aufzucht von *Spathius exarator.*

Der gemeine Nagekäfer (*Anobium punctatum*) ist ein Holzschädling, der allgemein auch unter dem Namen Holzwurm bekannt ist. Holzwürmer richten durch die Zerstörung von verarbeitetem Holz in Räumen mit hoher Luftfeuchtigkeit, vor allem auf Dachböden und in Kirchen, einen hohen wirtschaftlichen Schaden an. Dabei kann ein aktiver Holzwurmbefall durch Ausfluglöcher an den Holzoberflächen sowie durch Bohrmehlhäufchen, die sich unter den Ausfluglöchern bilden, erkannt werden. In der Vergangenheit wurden verschiedene Methoden der Bekämpfung von Holzwürmern vorgeschlagen, beispielsweise die Behandlung mit Insektiziden, die thermische Behandlung und auch die Begasung mit Stickstoff.

Ein neuer Ansatz zur Bekämpfung von *Anobium punctatum* ist die Verwendung von Nützlingen, also anderen Lebewesen, die Parasitoiden des *Anobium punctatum* sind. Ein geeigneter Gegenspieler des gemeinen Nagekäfers ist die Brackwespenart *Spathius exarator,* die als häufigster Parasitoid von *Anobium punctatum* beschrieben wird. Um einen derartigen Nützling effektiv einsetzen zu können, sind größere, schneller verfügbare Nutzpopulationen von *Spathius exarator* erforderlich. Im Stand der Technik ist jedoch noch nicht bekannt, wie die Brackwespenart *Spathius exarator* im Labor zur Eiablage zu bewegen ist oder gar eine stabile Massenzucht aufzubauen ist.

Die Druckschrift EP-A-1733617 offenbart die Bekämpfung von Anobium punctatum (Nagekäfer) mit Lagererwespen. Die Druckschrift US-A-3786594 offenbart ein Verfahren zur Aufzucht von Schlupfwespen mit einer Blattläuse aufweisenden Wirtspopulation.

AT 005240 U1 beschreibt lediglich eine Schlupfwespendose, die die Förderung der Schlupfwespenpopulation in einem natürlichen Lebensraum von Schlupfwespen erlaubt. Ein derartiges Vorgehen lässt sich jedoch nicht auf eine Labortätigkeit übertragen und bietet keinen Weg, eine Massenzucht von *Spathius exaratorzu* ermöglichen.

Der Erfindung liegt daher die Aufgabe zugrunde, eine Möglichkeit zur Aufzucht von *Spathius exarator* anzugeben, die eine Massenzucht dieser Nützlinge erlaubt.

Zur Lösung dieser Aufgabe ist erfindungsgemäß eine Zusammenstellung zur Aufzucht von *Spathius exarator* vorgesehen, aufweisend
- eine Aufzuchtpopulation von *Spathius exarator,*
- eine Wirtspopulation und
- einen Träger für die Aufzuchtpopulation und die Wirtspopulation, welche sich dadurch auszeichnet, dass die Wirtspopulation Larven von *Stegobium paniceum* und/oder *Lasioderma serricorne* aufweist und der Träger ausgetrocknetes Backwerk, insbesondere Brot, umfasst.

Die Verwendung von Holzwürmern, also *Anobium punctatum,* selbst eignet sich, wie im Rahmen der vorliegenden Erfindung festgestellt wurde, nicht zur Massenzucht von *Spathius exarator,* nachdem sich der Wirt nur äußerst langsam entwickelt, wobei zusätzlich zu bedenken ist, dass *Spathius exarator* ein Parasitoid des *Anobium punctatum* ist, mithin den Tod des Wirts zur Folge hat. Nach langwierigen Forschungen wurde von den Erfindern festgestellt, dass sich die mit *Anobium punctatum* verwandten Brotkäfer (*Stegobium paniceum*) und Tabakkäfer (*Lasioderma serricorne*) als Ersatzwirte verwenden lassen. Diese Käfer haben den Vorteil, dass sie, im Gegensatz zu *Anobium punctatum,* wesentlich einfacher unter Laborbedingungen zu züchten sind, da sie eine verhältnismäßig kurze Generationszeit, nämlich drei bis vier Generationen pro Jahre bei Zimmertemperatur, haben.

Allerdings hat sich auch gezeigt, dass sich weder freie Larven der Brotkäfer und Tabakkäfer noch Larven der Brotkäfer und Tabakkäfer in Holz als Wirtspopulation eignen, nachdem eine hohe Mortalität vorliegt. In Forschungen hat sich jedoch ergeben, dass hartes, altes Gebäck, mithin Backwerk, als Träger für die Wirtspopulation geeignet ist und auch durch die *Spathius exarator* akzeptiert wird. Mithin sieht die Erfindung vor, *Stegobium paniceum* und/oder *Lasioderma serricorne* als Wirtspopulation auf einem als ausgestrocknetes Backwerk ausgebildeten Träger der Aufzuchtpopulation von *Spathius exarator* zur Verfügung zu stellen, wobei sich bei Experimenten gezeigt hat, dass die Akzeptanz der Ersatzwirte durch die *Spathius exarator* äußerst hoch ist und sich sehr gute Vermehrungsraten erzielen lassen. Es wird also eine schneller und geeigneter herstellbare Ersatzfortpflanzungsumgebung für die Brackwespen gezielt erzeugt.

Auf diese Weise ist es somit erstmals möglich, *Spathius exarator* in größeren Mengen zu züchten, um sie beispielsweise als Nützlinge gegen Holzwürmer einzusetzen.

Zweckmäßig ist es, wenn das Backwerk wenigstens drei Tage, insbesondere in Luft, getrocknet ist. Auf diese Weise werden für den Träger Eigenschaften erreicht, die es sowohl den *Stegobium paniceum* und/oder den *Lasioderma serricorne* erlauben, sich in das Backwerk, insbesondere das Brot, einzubohren, auf der anderen Seite aber von den *Spathius exarator* auch als Fundort für potentielle Wirte akzeptiert wird. Es ist jedoch alternativ auch möglich, das Backwerk, insbesondere in einem Backofen, bei 80°C für 40 bis 50 Minuten, beispielsweise 45 Minuten, zu trocknen. Bevorzugt hat das getrocknete Backwerk eine verbleibende Materialfeuchte zwischen 10-13%.

Als besonders zweckmäßig hat es sich erwiesen, wenn das Backwerk wenigstens teilweise ausgehöhltes Baguette ist. Es wurde festgestellt, dass zu weiches oder zu raues Brot weniger geeignet ist, nachdem die *Spathius exarator* nicht mit ihrem Legestachel hineingelangen können oder der Legestachel gar steckenbleibt. Ein Baguette mit einer recht glatten Oberfläche hat sich als gut geeignet herausgestellt. Um die Baguettes als Träger zu präparieren, können handelsübliche Baguettes geeignet aufgeschnitten werden, wonach bevorzugt das weiche Weißbrot im Inneren entfernt und verworfen wird. Ein 60 cm langes Baguette kann beispielsweise in drei jeweils 20 cm lange Teile zerteilt werden, welche dann längs zur Bildung von je zwei Baguettestücken aufgeteilt werden. Die insbesondere ausgehöhlten Baguettestücke werden für drei bis vier Tage an Luft getrocknet, woraufhin auf ein derartiges getrocknetes Baguettestück die Larven verteilt werden, welche sich nach ungefähr zwei bis drei Tagen unter die harte Ringe gebohrt haben. Dann können die *Spathius exarator* hinzugefügt werden. Die verbleibende Wanddicke eines derartigen Baguettestücks oder allgemein des Backwerks kann zweckmäßigerweise 0,8 bis 1 cm betragen. Damit wird sichergestellt, dass die *Spathius exarator* mit ihrem Legestachel alle eingebohrten Larven erreichen können. Die sich ergebende Schalenform beim Aushöhle der Baguettestücke hat zudem den Vorteil, dass die eingesetzten Larven weniger leicht herausfallen können.

Eine weitere Ausgestaltung der Zusammenstellung sieht vor, dass das Verhältnis der Mitglieder der Aufzuchtpopulation zu den Mitgliedern der Wirtspopulation im Bereich von 1:2 - 1:8 liegt, bevorzugt bei 1:6. Ein Verhältnis von 1:6 hat sich als besonders günstig herausgestellt.

Vorzugsweise weisen die Larven der Wirtspopulation eine Mindestgröße von 2 mm auf. Derartige Larven können von einem Zuchtansatz beispielsweise durch Sieben extrahiert werden. Eine Größe der Larven von 2 mm deutet an, dass sie zum einen in der Lage sind, sich unter die harte Rinde des Backwerks, insbesondere des Brotes, zu bohren, zum anderen ist ihre Größe für die Fortpflanzung der *Spathius exarator* äußerst geeignet.

Zweckmäßigerweise kann die Zusammenstellung ferner eine Nahrungsquelle für die *Spathius exarator* umfassen, insbesondere ein mit einer Honig-Wasser-Lösung versetztes Wattestück. Um die Ernährung der Aufzuchtpopulation und/oder gegebenenfalls gezüchteter *Spathius exaratorzu* sichern, ist mithin eine Nahrungsquelle als Teil der Zusammensetzung vorgesehen, wobei beispielsweise ein Wattestückchen mit 20%iger Honig-Wasser-Lösung hinzugegeben werden kann, welches beispielsweise alle zwei bis drei Tage erneuert werden kann.

Neben der Zusammenstellung betrifft die Erfindung auch eine Aufzuchtanordnung, umfassend ein Aufzuchtgefäß, insbesondere auch Plastik, mit einer erfindungsgemäßen Zusammenstellung. Als Aufzuchtgefäß bietet sich insbesondere ein Faunarium an, welches in verschiedenen Größen bereits auf dem Markt erhältlich ist. Sollten auf dem Markt erhältliche Faunarien bzw. Aufzuchtgefäße zu große Lüftungsritzen, beispielsweise in einem Deckel, aufweisen, kann beispielsweise vorgesehen sein, dass ein handelsübliches Spültuch oder sonstiges Textil zwischen dem Deckel und dem restlichen Behälter vorgesehen wird. Sämtliche Ausführungen zur erfindungsgemäßen Zusammenstellung lassen sich analog auf die erfindungsgemäße Aufzuchtanordnung übertragen, mit welcher auch die genannten Vorteile erreicht werden können. Insbesondere ist eine derartige Aufzuchtanordnung zur Platzierung in einem zur Massenzucht von *Spathius exarator* vorgesehenen Labor geeignet.

Die Erfindung betrifft ebenso ein Verfahren zur Aufzucht von *Spathius exarator* unter Verwendung einer Zusammenstellung oder einer Aufzuchtanordnung der erfindungsgemäßen Art. Dabei werden mithin eine Nutzung der Wirtspopulation durch die *Spathius exarator* begünstigende Umgebungsbedingungen hergestellt. Sämtliche Ausführungen zur erfindungsgemäßen Zusammenstellung und zur erfindungsgemäßen Aufzuchtanordnung lassen sich analog auf das erfindungsgemäße Verfahren übertragen, was selbstverständlich, soweit anwendbar, auch umgekehrt gilt. Mithin lässt sich das erfindungsgemäße Verfahren insbesondere auch zur Massenzucht von *Spathius exarator* einsetzen.

Wird die Zusammenstellung bzw. die Aufzuchtanordnung erzeugt, ist mithin zuerst der Träger vorzubereiten, welcher bevorzugt, wie dargelegt wurde, aus einem handelsüblichen Baguette gebildet wird, welches bevorzugt für mindestens drei, insbesondere drei bis vier, Tage getrocknet wird. Dann werden auf das getrocknete Baguettestück, allgemein gesagt das getrocknete Backwerk, zunächst Larven der *Stegobium paniceum* und/oder der *Lasioderma serricorne* verteilt, die bevorzugt eine Mindestgröße von 2 mm aufweisen. Damit sich die Larven der Käfer unter die harte Rinde bohren können und somit Vorbereitungen zur Aufzucht von *Spathius exarator* getroffen sind, wird vorzugsweise die Aufzuchtpopulation frühestens 48 Stunden und/oder spätestens 72 Stunden nach dem Zusammenbringen der Wirtspopulation und des Trägers hinzugefügt. Es wurde festgestellt, dass sich die Larven nach zwei bis drei Tagen entsprechend eingebohrt haben.

Es kann ferner vorgesehen sein, dass nach einer Fortpflanzungszeit, insbesondere 18 - 24 Tagen, die Aufzuchtpopulation von der Zusammenstellung entfernt wird. Nach einer Fortpflanzungszeit, beispielsweise drei Wochen, werden die letzten noch lebenden *Spathius exarator* der Aufzuchtpopulation aus der Zusammenstellung, insbesondere aus dem Aufzuchtgefäß, abgefangen. Etwa eine Woche später schlüpfen dann die frischen *Spathius exarator.*

Dabei kann im Übrigen vorgesehen sein, dass bei Verwendung eines Aufzuchtgefäßes geschlüpfte *Spathius exarator* mit einem insbesondere zum Lagern der *Spathius exarator* ausgebildeten Saugrohr abgesaugt werden. Es kann mithin ein Saugrohr (Exhaustor) verwendet werden, um die neu gezüchteten *Spathius exarator* abzufangen, wobei es denkbar ist, dass das Saugrohr im Folgenden auch zur Aufbewahrung und zum Transport genutzt wird. Beispielsweise können die geschlüpften *Spathius exarator* in ein Gebäude mit von Holzwürmer befallenen Holzbestandteilen oder -gegenständen ausgebracht werden und dergleichen.

Erfindungsgemäß werden die Umgebungsbedingungen der Zusammenstellung so gewählt, dass die Fortpflanzung der *Spathius exarator* möglichst begünstigt wird. In diesem Zusammenhang sieht eine besonders bevorzugte Ausgestaltung der Erfindung vor, dass zur Herstellung von die Nutzung der Wirtspopulation verbessernden Umgebungsbedingungen eine Temperatur der Zusammenstellung in einem Bereich von 20 - 25° C, bevorzugt 23° C, gehalten wird und/oder eine Luftfeuchtigkeit von 50 - 70 %, bevorzugt 60 %, hergestellt wird, und/oder ein Licht-Dunkelheit-Zyklus mit längeren Lichtphasen als Dunkelheitsphasen verwendet wird, wobei bevorzugt die Lichtphasen 18 Stunden und die Dunkelheitsphasen 6 Stunden andauern. Es werden mithin für die Aufzucht durchgehend konstante Laborbedingungen hergestellt, wobei sich als eine gut geeignete Wahl eine Temperatur von 23° C, eine Luftfeuchtigkeit von 60 % und sogenannte Langtagbedingungen erwiesen haben, bei denen 18 Stunden Licht und 6 Stunden Dunkelheit (L18:D6) verwendet werden. Hierdurch lässt sich der Aufzuchterfolg weiter verbessern.

Während die Aufzuchtpopulation beispielsweise aus entsprechend geeigneten Eklektoren (Schlupfanlagen) gewonnen werden kann, aber auch frei eingefangen werden kann oder schließlich, wenn das Verfahren bereits durchgeführt wurde, aus dem Verfahrensergebnis abgetrennt werden kann, hat sich als problematisch erwiesen, dass die *Spathius exarator* Schädlinge, insbesondere Milben, aufweisen können, die den Aufzuchterfolg negativ beeinträchtigen können. Daher kann in einer vorteilhaften Weiterbildung des Verfahrens vorgesehen sein, dass die *Spathius exarator* der Aufzuchtpopulation vor Einsatz in der Zusammenstellung gegen Milben behandelt werden, insbesondere für 11 - 13 Stunden mit einer 1 %igen Beta-Naphthol-Lösung in Ethanol, welche bevorzugt auf einem Filterpapier in einem Quarantäne-Behälter eingebracht wird. Denkbar ist es also beispielsweise, die *Spathius exarator,* die als Aufzuchtpopulation verwendet werden, in einen Quarantäne-Zuchtbehälter zu setzen und für beispielsweise 12 Stunden mit 1 % Beta-Naphthol gegen Milben zu behandeln. Das Beta-Naphthol kann dabei in Ethanol gelöst sein, wobei bevorzugt eine bestimmte Menge der Lösung auf ein Filterpapier geträufelt wird, welches dann getrocknet und in den Quarantäne-Zuchtbehälter eingebracht wird. Nach einer bestimmten Behandlungszeit, beispielsweise 12 Stunden, können die *Spathius exarator* aus dem Quarantäne-Zuchtbehälter entnommen und in der Zusammenstellung eingesetzt werden, wodurch ein Milbenbefall und mithin eine negative Auswirkung auf die Aufzucht vermieden wird.

Die Wirtspopulation kann in einem Zuchtansatz erzeugt werden. Wie bereits erwähnt, werden Brotkäfer und/oder Tabakkäfer, genauer deren Larven, verwendet, die aufgrund einer verhältnismäßig kurzen Generationszeit gut unter Laborbedingungen zu züchten sind. Konkret kann vorgesehen sein, dass in dem Zuchtansatz ein Nährsubstrat mit 33 % Paniermehl, 33 % Hartweizengrieß, 33 % Weichweizengrieß und 1 % Glycerin verwendet wird. Ein derartiger Zuchtansatz kann beispielsweise in einem Glas aufbewahrt werden, welches durch ein luftdurchlässiges Stück Stoff und ein Gummiband abgedichtet wird. Nach einer bestimmten Zeit, beispielsweise 6 Wochen, kann eine Kontrolle der Zuchtansätze erfolgen, wobei dann, wenn sich Larven einer geeigneten Größe, wie beschrieben bevorzugt 2 mm oder größer, zeigen, diese extrahiert werden können.

Hierzu kann vorgesehen sein, dass zur Auswahl der Larven für die Wirtspopulation die Larven des Zuchtansatzes mit einem Sieb eines Maschendurchmessers von 2 mm gesiebt und die nicht das Sieb durchquerenden Larven als Wirtspopulation verwendet werden. Sobald also in dem Zuchtansatz geeignet große Larven ersichtlich sind, wird der Zuchtansatz durch ein Sieb mit einem Maschendurchmesser von 2 mm gesiebt. Dabei bleiben ausschließlich die größeren Larven und gegebenenfalls einige Puppen in dem Sieb zurück. Diese Larven werden nun als Wirtspopulation zur Aufzucht der *Spathius exarator* verwendet, während die kleineren Larven, die durch das Sieb gerutscht sind, weiterhin kultiviert werden können. Insbesondere kann vorgesehen sein, dass drei Abfolgen von Sieb- und Kultivierungsvorgängen erfolgen, wobei die Siebvorgänge jeweils um eine Woche beabstandet sind. Das verbliebene Nährsubstrat kann auch weiterverwertet werden, wenn zu diesem 1 % Glycerin gegeben wird und es bei -20° C gelagert wird, woraufhin das Nährsubstrat nach einer Woche wieder für neue Zuchtansätze verwendet werden kann.

Weitere Vorteile und Einzelheiten der vorliegenden Erfindung ergeben sich aus den im Folgenden beschriebenen Ausführungsbeispielen sowie anhand der Zeichnung. Dabei zeigen:
- Fig. 1: eine erfindungsgemäße Aufzuchtanordnung, und
- Fig. 2: ein als Träger verwendetes Baguettestück.

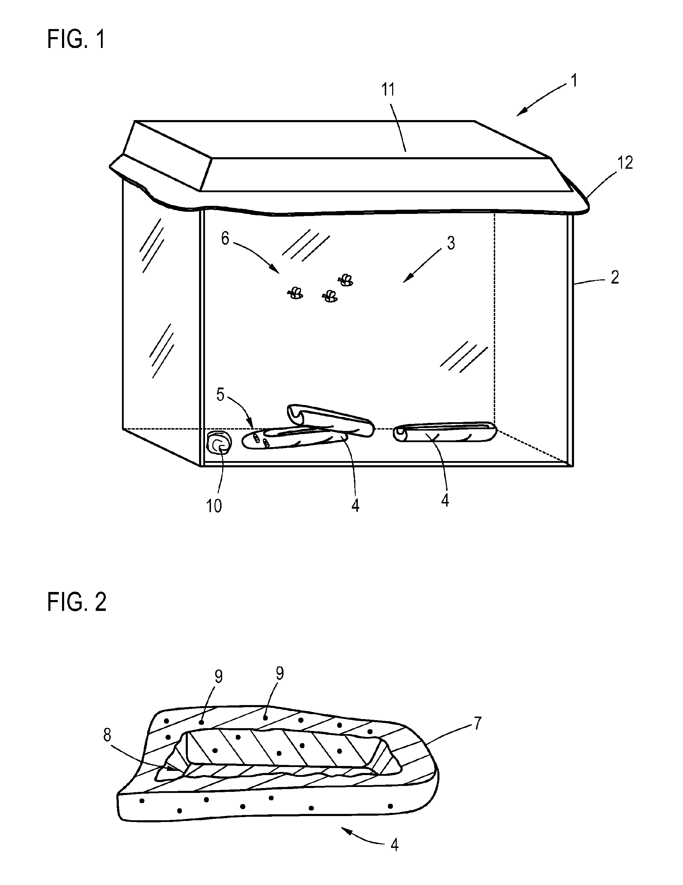

Fig. 1 zeigt in einer Prinzipskizze eine Aufzuchtanordnung 1, die zur Aufzucht der Brackwespenart *Spathius exarator* verwendet werden kann. In einem Aufzuchtgefäß 2, hier einem Faunarium, ist eine Zusammenstellung 3 angeordnet, die ohne die Verwendung des üblichen Wirts der *Spathius exarator,* nämlich des *Anobium punctatum*, eine Umgebung bietet, in der sich *Spathius exarator* fortpflanzen.

Hierzu umfasst die Zusammenstellung 3 einen Träger 4, nämlich ein drei bis vier Tage getrocknetes Baguettestück, in das sich eine Wirtspopulation 5, bestehend aus Larven von *Stegobium paniceum* und/oder *Lasioderma serricorne,* also Brotkäfern und Tabakkäfern, eingebohrt haben. Ferner ist eine Aufzuchtpopulation 6 von *Spathius exarator* vorgesehen.

Der Träger 4, gebildet durch das Baguettestück 7, ist in Fig. 2 näher gezeigt. Es handelt sich um ein Stück eines klassischen Baguettes, welches in seinem Inneren 8 von dem weichen Weißbrotanteil weitgehend befreit wurde und sodann drei bis vier Tage an der Luft getrocknet wurde. Werden nun Larven der Wirtspopulation 5 auf das Baguettestück 7 verteilt, bohren sich diese in das Baguettestück 7 ein, was in Fig. 2 an den Löchern 9, welche teilweise gezeigt sind, erkenntlich ist.

Durch nicht näher gezeigte, aber allgemein bekannte technische Mittel werden innerhalb der Aufzuchtanordnung 1, also konkret innerhalb des Aufzuchtgefäßes 2, Umgebungsbedingungen aufrechterhalten, die die Nutzung des Ersatzwirts, also der Larven des Brotkäfers und des Tabakkäfers, durch die *Spathius exarator* begünstigen und somit die Fortpflanzung verbessern. Vorliegend wird eine Temperatur von 23° C bei einer Luftfeuchtigkeit von 60 % aufrechterhalten; zudem wird die Zusammenstellung 3 18 Stunden lang Licht ausgesetzt, 6 Stunden lang der Dunkelheit.

Ein konkretes Beispiel zur Aufzucht von *Spathius exarator* unter Verwendung der in Fig. 1 und Fig. 2 gezeigten Umgebung soll nun näher dargelegt werden. Bevor der eigentliche Aufzuchtvorgang beginnt, wird die Aufzuchtpopulation 6 von *Spathius exarator,* die aus Eklektoren oder sonstigen Quellen stammen kann, zunächst gegen Milben behandelt. Hierzu werden die *Spathius exarator* in einen Quarantäne-Zuchtbehälter gesetzt und für 12 Stunden mit Beta-Naphthol gegen Milben behandelt. Eine 1 %ige Beta-Naphthol-Lösung in Ethanol wird hergestellt, eine bestimmte Menge, beispielsweise 5 ml, der Lösung wird auf ein Filterpapier geträufelt, welches nach Trocknung in den Quarantäne-Zuchtbehälter gelegt wird. Nach Ablauf der 12 Stunden werden die *Spathius exarator* aus dem Quarantäne-Zuchtbehälter benommen und können für die Aufzucht eingesetzt werden.

Wie bereits beschrieben wurde erkannt, dass die Ersatzwirte Brotkäfer und Tabakkäfer, welche unter Laborbedingungen wesentlich einfacher als *Anobium punctatum* zu züchten sind, in getrocknetem Backwerk von den *Spathius exarator* zur Fortpflanzung akzeptiert werden. Es wird mithin eine künstliche Umgebung für die Aufzuchtpopulation 6 bereitgestellt, in der eine Fortpflanzung und insbesondere eine Massenzucht realisiert werden kann. Hierzu ist zunächst die Wirtspopulation 5 heranzuziehen.

Dazu wird ein Nährsubstrat verwendet, im vorliegenden Beispiel eine Mischung aus 33 % Paniermehl, 33 % Hartweizengrieß, 33 % Weichweizengrieß und 1 % Glycerin. Ein Zuchtansatz für die Wirtspopulation 5 in einem Zuchtglas beinhaltet im hier gezeigten Beispiel 100 ml Nährsubstrat und rund 100 frisch geschlüpfte Käfer. Das Zuchtglas wird mit einem luftdurchlässigen Stück Stoff und einem Gummiband abgedichtet. Jeden zweiten Tag werden zwei neue Zuchtansätze angesetzt und bei den Laborbedingungen kultiviert, die auch später innerhalb des Aufzuchtgefäßes 2 herrschen, mithin bei 23° C, einer Luftfeuchtigkeit von 60 % und Langtagbedingungen.

Nach etwa 6 Wochen werden die Zuchtansätze kontrolliert. Zeichnen sich Larven von 2 mm oder größer am Boden des Zuchtglases ab, wird der Zuchtansatz durch ein Sieb mit einem Maschendurchmesser von 2 mm gesiebt. Dabei bleiben ausschließlich die größeren Larven und gegebenenfalls einige Puppen im Sieb zurück. Diese Larven werden für die Aufzucht der *Spathius exarator* als Wirtspopulation 5 verwendet, während die kleineren Larven durch das Sieb rutschen und weiterhin kultiviert werden. Nach drei Sieb- und Kultivierungsrunden im Abstand von jeweils einer Woche wird zu dem übriggebliebenen Nährsubstrat 1 % Glycerin gegeben und es wird bei -20° C eingelagert, so dass es nach etwa einer Woche wieder für neue Zuchtansätze verwendet werden kann.

Die Zucht der *Spathius exarator* erfolgt dann auf den präparierten Baguettestücken 7, bei denen, wie beschrieben wurde, das weiche Weißbrot im Inneren 8 entfernt wird, wonach die ausgehöhlten Baguettestücke 7 für drei bis vier Tage getrocknet werden, so dass sich idealerweise eine Materialfeuchte von 10 bis 13% ergibt. Die Wanddicke wird so gewählt, dass sie zwischen 0.8 und 1 cm beträgt. Werden handelsübliche Baguettes, beispielsweise der Länge 60 cm, in drei Teile aufgeteilt und aufgeschnitten, wobei die oberen Hälften auch verworfen werden können, können auf ein getrocknetes Baguettestück 7 beispielsweise 150 Käferlarven einer Mindestgröße von 2 mm verteilt werden. Nach 48 - 72 Stunden kann davon ausgegangen werden, dass sich die Käferlarven der Wirtspopulation 5 unter die harte Rinde gebohrt haben, so dass die Baguettestücke 7 den in Fig. 2 gezeigten Zustand erreicht haben und für die Aufzucht eingesetzt werden können.

Eine beispielhafte Zusammenstellung 3 (Aufzuchtansatz) in dem Aufzuchtgefäß 2 kann beispielsweise vier Baguettestücke 7 und etwa 100 *Spathius exarator,* männliche und weibliche Exemplare gemischt, als Aufzuchtpopulation 6 enthalten, so dass insgesamt etwa 100 *Spathius exarator* auf 600 Larven kommen; ein Verhältnis von 1:6. Zur Versorgung der *Spathius exaratorwird,* vgl. Fig. 1, ein Wattestückchen 10 mit einer 20 %igen Honig-Wasser-Lösung hinzugegeben, welches alle 48 bis 72 Stunden erneuert wird.

Nach drei Wochen werden dann die letzten, noch lebenden *Spathius exarator* der Aufzuchtpopulation 6 aus dem Aufzuchtgefäß 2 abgefangen, woraufhin etwa eine Woche später die frischen, gezüchteten *Spathius exarator* schlüpfen, welche dann aus dem Aufzuchtgefäß 2 mit einem Saugrohr abgesaugt werden können. In Experimenten wurden aus einem derartigen Aufzuchtansatz wie gerade beschrieben etwa 500 *Spathius exarator* gewonnen.

Zur Vervollständigung wird darauf hingewiesen, dass vorliegend ein Faunarium Kunststoff verwendet wurde, dessen Abmessungen etwa 23 x 15 x 16,5 cm betragen. Um zu vermeiden, dass die Tiere nicht aus den Lüftungsritzen des Deckels 11 entkommen, wurde ein Tuch 12, hier ein handelsübliches Spültuch, zwischen Deckel 11 und dem restlichen Aufzuchtgefäß 2 eingeklemmt.

## Patentansprüche

1. Zusammenstellung (3) zur Aufzucht von *Spathius exarator,* aufweisend
- eine Aufzuchtpopulation (6) von *Spathius exarator,*
- eine Wirtspopulation (5) und
- einen Träger (4) für die Aufzuchtpopulation (6) und die Wirtspopulation (5),
wobei die Wirtspopulation (5) Larven von *Stegobium paniceum* und/oder *Lasioderma serricorne* aufweist und der Träger (4) ausgetrocknetes Backwerk, insbesondere Brot, umfasst.

2. Zusammenstellung nach Anspruch 1, **dadurch gekennzeichnet, dass** das Backwerk wenigstens drei Tage, insbesondere in Luft, getrocknet ist.

3. Zusammenstellung nach Anspruch 1 oder 2, **dadurch gekennzeichnet**, das Verhältnis der Mitglieder der Aufzuchtpopulation (6) zu den Mitgliedern der Wirtspopulation (5) im Bereich von 1:2 bis 1:8 liegt, bevorzugt bei 1:6.

4. Zusammenstellung nach einem der vorangehenden Ansprüche, **dadurch gekennzeichnet, dass** die Larven eine Mindestgröße von 2 mm aufweisen.

5. Zusammenstellung nach einem der vorangehenden Ansprüche, **dadurch gekennzeichnet, dass** sie ferner eine Nahrungsquelle für die *Spathius exarator* umfasst, insbesondere ein mit einer Honig-Wasser-Lösung versetztes Wattestück (10).

6. Aufzuchtanordnung (1), umfassend ein Aufzuchtgefäß (2), insbesondere aus Kunststoff, mit einer Zusammenstellung (3) nach einem der vorangehenden Ansprüche.

7. Verfahren zur Aufzucht von *Spathius exarator* unter Verwendung einer Zusammenstellung (3) nach Anspruch 1 bis 5 oder einer Aufzuchtanordnung (1) nach Anspruch 6.

8. Verfahren nach Anspruch 7, **dadurch gekennzeichnet, dass** die Aufzuchtpopulation (6) frühestens 48 Stunden und/oder spätestens 72 Stunden nach dem Zusammenbringen der Wirtspopulation (5) und des Trägers (4) hinzugefügt wird.

9. Verfahren nach Anspruch 7 oder 8, **dadurch gekennzeichnet, dass** nach einer Fortpflanzungszeit, insbesondere 18-24 Tagen, die Aufzuchtpopulation (6) von der Zusammenstellung (3) entfernt wird.

10. Verfahren nach einem der Ansprüche 7 bis 9, **dadurch gekennzeichnet, dass** bei Verwendung eines Aufzuchtgefäßes (2) geschlüpfte *Spathius exarator* mit einem insbesondere zum Lagern der *Spathius exarator* ausgebildeten Saugrohr abgesaugt werden.

11. Verfahren nach einem der Ansprüche 7 bis 10, **dadurch gekennzeichnet, dass** zur Herstellung von die Nutzung der Wirtspopulation verbessernden Umgebungsbedingungen eine Temperatur der Zusammenstellung (3) in einem Bereich von 20 bis 25 °C, bevorzugt 23 °C, gehalten wird und/oder eine Luftfeuchtigkeit von 50-70%, bevorzugt 60%, hergestellt wird und/oder ein Licht-Dunkelheit-Zyklus mit längeren Lichtphasen als Dunkelheitsphasen verwendet wird, wobei bevorzugt die Lichtphasen 18 Stunden und die Dunkelheitsphasen 6 Stunden andauern.

12. Verfahren nach einem der Ansprüche 7 bis 11, **dadurch gekennzeichnet, dass** die *Spathius exarator* der Aufzuchtpopulation (6) vor Einsatz in der Zusammenstellung gegen Milben behandelt werden, insbesondere für 11-13 Stunden mit einer 1 %tigen beta-Naphtol-Lösung in Ethanol, welche bevorzugt auf einem Filterpapier in einen Quarantäne-Behälter eingebracht wird.

13. Verfahren nach einem der Ansprüche 7 bis 12, **dadurch gekennzeichnet, dass** die Wirtspopulation (5) in einem Zuchtansatz erzeugt wird.

14. Verfahren nach Anspruch 13, **dadurch gekennzeichnet, dass** zur Auswahl der Larven für die Wirtspopulation (5) die Larven des Zuchtansatzes mit einem Sieb eines Maschendurchmessers von 2mm gesiebt und die nicht das Sieb durchquerenden Larven als Wirtspopulation (5) verwendet werden.

## Claims

1. Arrangement (3) for breeding *Spathius exarator,* including
- a breeding population (6) of *Spathius exarator,*
- a host population (5) and
- a support (4) for the breeding population (6) and the host population (5),
where
the host population (5) displays larvae of *Stegobium paniceum* and/or *Lasioderma serricome* and the support (4) comprises dried baked goods, in particular bread.

2. Arrangement according to Claim 1, **characterized in that** the baked goods have been dried for at least three days, in particular in the air.

3. Arrangement according to Claim 1 or 2, **characterized in that** the ratio of the members of the breeding population (6) to the members of the host population (5) is in the range of from 1:2 to 1:8, preferably 1:6.

4. Arrangement according to one of the preceding claims, **characterized in that** the larvae have a minimum size of 2 mm.

5. Arrangement according to one of the preceding claims, **characterized in that** it furthermore comprises a feed source for the *Spathius exarator,* in particular a piece of cotton (10) treated with a honey-water solution.

6. Breeding assembly (1) comprising a breeding container (2), in particular made of plastic, with an arrangement (3) according to one of the preceding claims.

7. Method of breeding *Spathius exarator* using an arrangement (3) according to Claims 1 to 5 or breeding assembly (1) according to Claim 6.

8. Method according to Claim 7, **characterized in that** the breeding population (6) is added at the earliest 48 hours and/or at the latest 72 hours after combining the host population (5) and the support (4).

9. Method according to Claim 7 or 8, **characterized in that** the breeding population (6) is removed from the arrangement (3) after a reproduction time of in particular 18-24 days.

10. Method according to one of Claims 7 to 9, **characterized in that**, when using a breeding container (2), hatched *Spathius exarator* are removed by suction, using a suction tube especially designed for storing the *Spathius exarator.*

11. Method according to one of Claims 7 to 10, **characterized in that**, to establish environment conditions which improve the utilization of the host population, a temperature of the arrangement (3) is maintained in a range of from 20 to 25°C, preferably 23°C, and/or an atmospheric humidity of 50-70%, preferably 60%, is established and/or a light/dark cycle with light phases longer than the dark phases is used, with the light phases preferably having a duration of 18 hours and the dark phases a duration of 6 hours.

12. Method according to one of Claims 7 to 11, **characterized in that**, before being used in the arrangement, the *Spathius exarator* of the breeding population (6) are treated against mites, in particular for 11-13 hours with a 1% strength beta-naphthol solution in ethanol, which is, preferably on a filter paper, introduced into a quarantine container.

13. Method according to one of Claims 7 to 12, **characterized in that** the host population (5) is generated in a breeding batch.

14. Method according to Claim 13, **characterized in that**, for selecting the larvae for the host population (5), the larvae of the breeding batch are screened with the screen of mesh size 2 mm and the larvae which do not pass through the screen are used as host population (5).

## Revendications

1. Composition (3) pour l'élevage de *Spathius exarator,* présentant
- une population d'élevage (6) de *Spathius exarator,*
- une population-hôte (5) et
- un support (4) pour la population d'élevage (6) et la population-hôte (5),
dans laquelle la population-hôte (5) présente des larves de *Stegobium paniceum* et/ou de *Lasioderma serricorne* et le support (4) comprend un produit de boulangerie séché, en particulier du pain.

2. Composition selon la revendication 1, **caractérisée en ce que** le produit de boulangerie est séché au moins trois jours, en particulier dans l'air.

3. Composition selon la revendication 1 ou 2, **caractérisée en ce que** le rapport des membres de la population d'élevage (6) aux membres de la population-hôte (5) se situe dans la plage de 1:2 à 1:8, de préférence à 1:6.

4. Composition selon l'une quelconque des revendications précédentes, **caractérisée en ce que** les larves présentent une grandeur minimum de 2 mm.

5. Composition selon l'une quelconque des revendications précédentes, **caractérisée en ce qu'**elle comprend en outre une source de nutriments pour le *Spathius exarator,* en particulier un morceau d'ouate (10) imbibé avec une solution de miel et d'eau.

6. Dispositif d'élevage (1), comprenant une enceinte d'élevage (2), en particulier en matière plastique, avec une composition (3) selon l'une quelconque des revendications précédentes.

7. Procédé pour l'élevage de *Spathius exarator* avec utilisation d'une composition (3) selon la revendication 1 ou d'un dispositif d'élevage (1) selon la revendication 6.

8. Procédé selon la revendication 7, **caractérisé en ce que** la population d'élevage (6) est introduite au plus tôt 48 heures et/ou au plus tard 72 heures après la mise en présence de la population-hôte (5) et du support (4).

9. Procédé selon la revendication 7 ou 8, **caractérisé en ce que** la population d'élevage (6) est retirée de la composition (3) après un temps de propagation, en particulier 18 - 24 jours.

10. Procédé selon l'une quelconque des revendications 7 à 9, **caractérisé en ce que**, en utilisant une enceinte d'élevage (2), les *Spathius exarator* éclos sont aspirés avec un tube aspirant configuré en particulier pour le dépôt des *Spathius exarator.*

11. Procédé selon l'une quelconque des revendications 7 à 10, **caractérisé en ce que**, pour créer des conditions ambiantes améliorant l'utilisation de la population-hôte, on maintient une température de la composition (3) dans une plage de 20 à 25°C, de préférence de 23°C et/ou on établit une humidité de l'air de 50-70 %, de préférence 60 %, et/ou on utilise un cycle lumière/obscurité avec des phases de lumière plus longues que les phases d'obscurité, dans lequel de préférence les phases de lumière durent 18 heures et les phases d'obscurité 6 heures.

12. Procédé selon l'une quelconque des revendications 7 à 11, **caractérisé en ce que** les *Spathius exarator* de la population d'élevage (6) sont traités contre les acariens avant leur utilisation dans la composition, en particulier pendant 11-13 heures avec une solution de bêta-naphtol 1 % dans l'éthanol, qui est introduite de préférence sur un papier filtre dans un récipient de quarantaine.

13. Procédé selon l'une quelconque des revendications 7 à 12, **caractérisé en ce que** la population-hôte (5) est produite dans une installation d'élevage.

14. Procédé selon la revendication 13, **caractérisé en ce que**, pour la sélection des larves destinées à la population-hôte (5), les larves de l'installation d'élevage sont triées avec un tamis présentant un diamètre de mailles de 2 mm et les larves qui ne traversent pas le tamis sont utilisées comme population-hôte (5).
